# EUROPEAN PATENT APPLICATION

(11) **EP 1 669 477 A1**
(43) Date of publication of application: **14.06.2006**
(21) Application number: 05017551.2
(22) Date of filing: 27.10.2000
(51) Int. Cl.: C23C 22/68, C23C 8/12, C23C 8/14, A61K 6/02, A61L 27/02, A61L 27/04, A61L 27/06, A61K 6/00

(54) **Method of forming an oxide film on a metallic member and method of cementing the metallic member**

(30) Priority: 29.10.1999 JP 30880999; 18.10.2000 JP 2000317987
(62) Divisional of application: 00123350.1
(71) Applicant: Matsumoto Dental University, Nagano 399-0781 (JP)
(72) Inventor: Ito, Michio, Shiojiri, Nagano 399-0742 (JP)
(74) Representative: Hering, Hartmut

(57) **Abstract**

In a method of fastening a metallic member to a fixing part, such as an affected part, a predetermined portion of the metallic member is oxidized on its surface by a hydrogen peroxide solution to form an oxide film. The metallic member is fixed to the fixing part at the oxidized predetermined portion by a cementing material. On the oxidized predetermined portion, silane may be applied before the metallic member is cemented by the cementing material.

## Description

This invention relates to a method of forming an oxide film on a surface of a metallic member to be cemented to a fixing site by the use of a cementing material and to a method of cementing the metallic member.

Traditionally, in the field of dental treatment, a dental article comprising a metallic member is fixed through a cementing material to an affected part of a patient as a fixing site. Such dental article comprising the metallic member may be an orthodontic bracket, a crown or a bridge as prosthetic material, or an inlay for conservative restoration.

As the cementing material, use is typically made of a dental cement. As a conventional cementing technique, the following methods are adopted in order to increase the adhesive strength between the metallic member and the dental cement.

In one cementing method, the surface of the metallic member is sandblasted to be polished. The metallic member with its surface sandblasted is placed on the affected part of the patient through the dental cement and then cemented by the dental cement.

In another cementing method, the metallic member to be cemented is heated in an electric furnace to form an oxide film on the surface of the metallic member. Then, the metallic member with the oxide film formed thereon is located on the affected part through the dental cement and cemented by the dental cement. In this method, the oxide film is firmly formed on the surface of the metallic member.

Before placing the metallic member on the affected part of the patient, it is necessary to partially remove the oxide film from the surface of the metallic member in an area except a cemented portion to be cemented to the dental cement and to polish the area into a mirror-finished surface.

On the other hand, an implant used in the dental field is heated in the electric furnace to be oxidized and then directly implanted in the affected part of the patient to be bonded to a living bone.

However, the metallic member requires a long polishing time to remove the oxide film as mentioned above. In addition, the metallic member may be deteriorated in quality because it is heated in the electric furnace to a high temperature to be oxidized.

It is therefore an object of this invention to provide a method of forming an oxide film on a metallic member, which is capable of avoiding the deterioration in quality of the metallic member as a result of heat treatment to form an oxide film and which is capable of easily forming the oxide film in a predetermined portion of the metallic member.

It is another object of this invention to provide a method of cementing a metallic member, which is capable of avoiding the deterioration in quality of the metallic member as a result of heat treatment to form the oxide film, which does not require a long polishing time, and which is capable of improving reliability and strength of cementing.

According to this invention, there is provided a method of forming an oxide film on the surface of a predetermined portion of a metallic member, wherein the surface of the predetermined portion is oxidized by a hydrogen peroxide solution to form the oxide film.

According to this invention, there is also provided a method of cementing a metallic member by forming an oxide film on the surface of a predetermined portion of the metallic member to be cemented and thereafter cementing the predetermined portion and a fixing site through a cementing material, wherein the surface of the predetermined portion is oxidized by a hydrogen peroxide solution before the predetermined portion and the fixing site are cemented through the cementing material.
Fig. 1 is a graph showing an adhesive or bonding strength according to a first embodiment of this invention with respect to titanium as a metallic member;
Fig. 2 is a graph similar to Fig. 1 but with respect to a gold-silver-palladium alloy as a metallic member;
Fig. 3 is a graph showing an adhesive strength according to a known technique with respect to titanium as a metallic member;
Fig. 4 is graph similar to Fig. 3 but with respect to a gold-silver-palladium alloy as a metallic member;
Fig. 5 is a graph showing an adhesive strength according to a second embodiment of this invention; and
Fig. 6 is a graph showing a bonding strength in case where the first embodiment of this invention is applied to an implant.

Now description will be made of a method of forming an oxide film on a metallic member and a method of cementing a metallic according to a first embodiment of this invention.

In the oxide film forming method of the first embodiment, the oxide film is formed by applying a hydrogen peroxide (H₂O₂) solution on the surface of a predetermined portion of the metallic member.

In the cementing method of the first embodiment, the oxide film is at first formed on a metallic member to be cemented. Specifically, the oxide film is formed by applying a hydrogen peroxide (H₂O₂) solution on the surface of a predetermined portion of the metallic member. Thereafter, the metallic member with the oxide film formed thereon is cemented through a cementing material to a fixing site of a patient as an affected part.

For example, in the dental field, the metallic member such as an orthodontic bracket, a crown or a bridge as a prosthetic material, or an inlay for conservative restoration is cemented to the affected part by the use of the cementing material. As the cementing material, use is made of a dental cement.

In order to apply the hydrogen peroxide solution on the surface of the predetermined portion of the metallic member, use may be made of cotton or sponge. The hydrogen peroxide solution is absorbed into the cotton or the sponge which is then brought into contact with the surface of the predetermined portion of the metallic member.

After the hydrogen peroxide solution is applied as mentioned above, the oxide film is formed on the surface of the predetermined portion of the metallic member after lapse of one or two minutes. Thereafter, the metallic member is fixed to the fixing site or the affected part of the patient by the use of the cementing material.

As the metallic member, use may be made of pure titanium (Ti), a titanium alloy, a gold-silver-palladium(Au-Ag-Pd) alloy, a silver (Ag) alloy, a cobalt-chromium (Co-Cr) alloy, a nickel-chromium (Ni-Cr-) alloy, a stainless steel, a platinium gold alloy, and a gold (Au) alloy.

By the above-mentioned oxide film forming method above, the oxide film similar to that obtained by oxidization in an electric furnace is formed on the surface of the metallic member. Therefore, it is possible to avoid the deterioration in quality of the metallic member as a result of the heat treatment in the electric furnace and to save a polishing time partially remove the oxide film. By the cementing method mentioned above, the adhesive strength between the metallic member and the cementing material is improved.

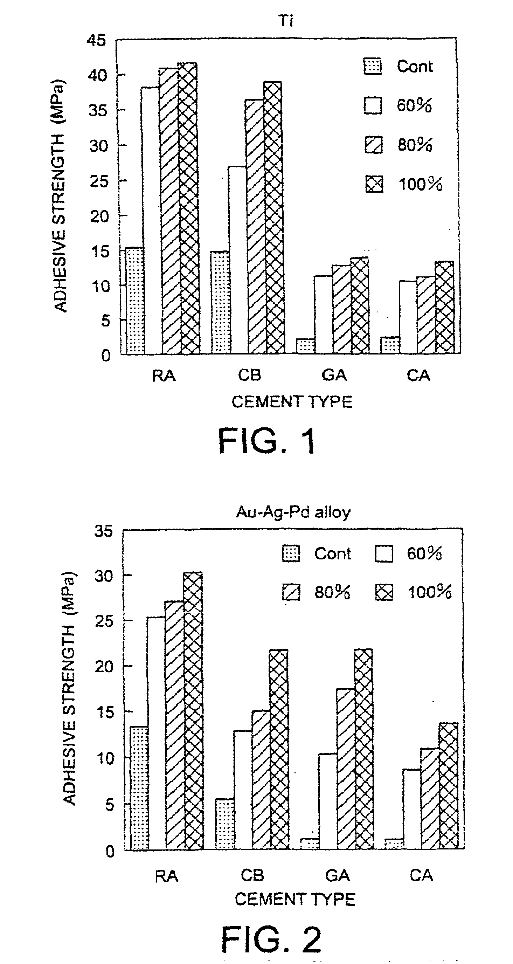

Referring to Figs. 1 through 4, description will hereafter be made of the adhesive strength (MPa) or bonding strength achieved and measured in an experimental test in which metallic plates are cemented by the use of four kinds of dental cements RA, CB, GA and Ca as cementing materials. The dental cements RA, CB, GA and CA are commercially available in the name of Super-Bond (manufactured by Sun Medical), Livcarbo (manufactured by GC), Fuji Bond (manufactured by GC), and FH Cement (manufactured by Nissin), respectively.

In the experimental test, the adhesive strength was measured in the following manner. At first, a plurality of pairs of metal plates of 10 mm square were prepared as metallic members. The metal plate pairs as samples were grouped into first through fourth groups. An oxide film was formed on each metal plate in the manner which will be described below. Then, the metal plates in each sample were cemented to each other by the use of a cementing material. Specifically, the dental cements RA, CB, GA and CA were used in the first through the fourth groups, respectively. Thereafter, one of the metal plates in each sample was given a load in a direction parallel to a plate plane to measure the adhesive strength (MPa: megapascal) against shearing force. In addition to the samples, for each of the first through the fourth groups, preparation was also made of a control (Cont) in which no oxide film was formed on the metal plates cemented to each other.

Referring to Fig. 1, a titanium (Ti) plate was used as each metal plate. The hydrogen peroxide solution was applied onto the surface of the titanium plate to form the oxide film thereon. Then, the titanium plates in each sample were cemented to each other by the use of the dental cements RA, CB, GA, and CA in the first through the fourth groups, respectively.

In the example being illustrated in Fig. 1, the hydrogen peroxide solution was used in three different concentrations in each of the first through the fourth groups. Specifically, a 34% hydrogen peroxide solution was used as a 100% starting solution. The starting solution was diluted to 60%, (34% x 60%), diluted to 80% (34 % x 80%), and not diluted (34% x 100%). As mentioned above, preparation was made of the control without oxide films, i.e., without the treatment by the hydrogen peroxide solution for each of the first through the fourth groups.

As is obvious from Fig. 1, in the control without the oxide film formed on the surface of the titanium plate, the adhesive strength was around 15 MPa (when RA and CB are used) and smaller than 3 MPa (when GA and CA are used). On the other hand, in the samples with the oxide films, the adhesive strength was greater than 38 MPa (RA), greaten than 27 MPa (CB), and greater than 10 MPa (GA and CA).

Referring to Fig. 2, a gold-silver-palladium (Au-Ag-Pd) alloy plate was used as each metal plate. The alloy plates were treated by the hydrogen peroxide solution, cemented to each other, and measured for the adhesive strength in the manner similar to that mentioned in conjunction with Fig. 1.

In the example being illustrated in Fig. 2, the hydrogen peroxide solution was used in three different concentrations in each of the first through the fourth groups, in the manner similar to that mentioned in conjunction with Fig. 1. As mentioned above, preparation was also made of control without the oxide film, i.e., without the treatment by the hydrogen peroxide solution for each of the first through the fourth groups.

As is obvious from Fig. 2, in the control without the oxide film formed on the surface of the Au-Ag-Pd alloy plate, the adhesive strength was smaller than 15 MPa (RA), around 5 MPa (CB), and smaller than 2 MPa (GA and CA). On the other hand, in the samples with the oxide films, the adhesive strength was greater than 25 MPa (RA), greaten than 12 MPa (CB), greater than 10 MPa (GA), and greater than 8 MPa (CA).

Furthermore, in order to prove the effect of this invention, a similar test was performed upon a conventional method of heating each of metal plates to form an oxide film on the surface thereof and cementing the metal plates to each other by the use of the dental cements RA, CB, GA, and CA.

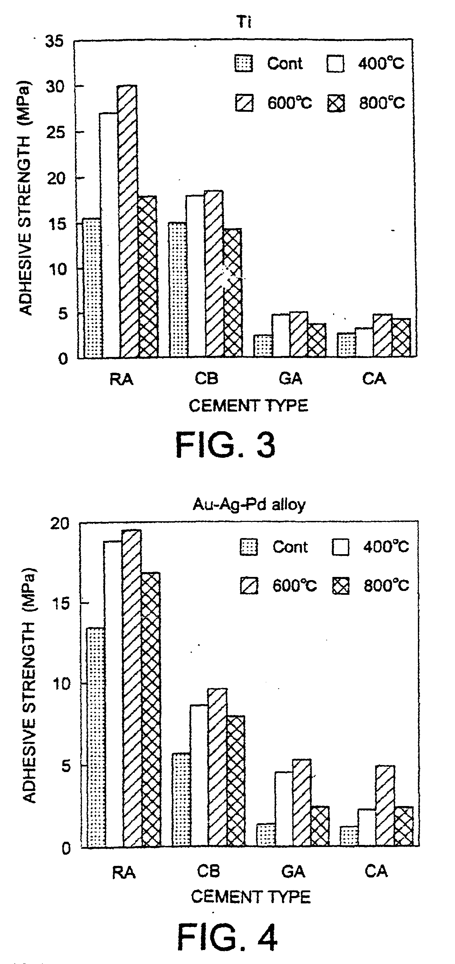

Referring to Fig. 3, a titanium plate was used as each metal plate. Each of the titanium plate was heated in an electric furnace to form the oxide film on the surface thereof. Thereafter, the titanium plates in each sample were cemented to each other by the use of the dental cements RA, CB, GA, and CA in first through fourth groups, respectively, and measured for the adhesive strength in the manner similar to that mentioned in conjunction with Fig. 1.

In the example being illustrated in Fig. 3, the titanium plate was heated in the electric furnace for 30 minutes at three different temperatures of 400°C, 600°C, and 800°C, respectively, in each of the first through the fourth groups. As mentioned above, preparation was also made of the control without the oxide films, i.e., without the heat treatment in the electric furnace for each of the first through the fourth groups.

As seen from Fig. 3, the adhesive strength was as small as 5 MPa or less with the dental cements GA and CA.

Referring to Fig. 4, a gold-silver-palladium (Au-AG-Pd) alloy plate was used as each metal plate. Each of Au-Ag-Pd alloy plates was heated in the electric fumace, cemented to each other, and measured for the adhesive strength in the manner similar to that mentioned in conjunction with Fig. 3.

In the example being illustrated in Fig. 4, the Au-Ag-Pd alloy plate was heated in the electric furnace for 30 minutes at three different temperatures of 400°C, 600°C, and 800°C, respectively, in each of the first through the fourth groups, in the manner similar to that mentioned in conjunction with Fig. 3. As mentioned above, preparation was also made of the control without the oxide films, i.e., without the heat treatment in the electric furnace for each of the first through the fourth groups.

As seen from Fig. 4, the adhesive strength was as small as 5 MPa or less with the dental cements GA and CA.

Comparison will be made between the adhesive strengths between the titanium plates in the first embodiment in Fig. 1 and the conventional method in Fig. 3. It is needless to say that the adhesive strength of the control without the oxide film is unchanged between Figs. 1 and 3 for each of the dental cements RA, CB, GA, and CA. For the samples with the oxide films, it has been confirmed that the treatment by the hydrogen peroxide solution in the first embodiment achieved a greater adhesive strength than that achieved by the heat treatment in the electric furnace in the conventional method for each of the dental cements RA, CB, GA, and CA.

Likewise, comparison will be made between the adhesive strengths between the Au-Ag-Pd alloy plates in the first embodiment in Fig. 2 and the conventional method in Fig. 4. For the samples with the oxide films, it has been confirmed that the treatment by the hydrogen peroxide solution in the first embodiment achieved a greater adhesive strength than that achieved by the heat treatment in the electric furnace in the conventional method for each of the dental cements RA, CB, GA, and CA. In particular, the adhesive strength achieved in the groups using the dental cements RA and CB is sufficient for use in the affected part of the patient in the dental field.

From the experimental tests, it is understood that, when the oxide film is formed on the surface of the metallic member according to the oxide film forming method of the above-mentioned embodiment, wettability between the cementing material (cement) and the metallic member (metal plate) is improved. Furthermore, according to the cementing method of the above-mentioned embodiment, the adhesive strength is improved by chemical bond between the oxide film and the cementing material.

In the experimental test, the titanium plate and the gold-silver-palladium alloy plate were used as the metallic member. It has also be confirmed that, in other metallic members such as stainless steel, a titanium alloy, a platinum gold alloy, a silver alloy, and a gold alloy, the adhesive strength is substantially improved as compared with the case where the oxide film is formed by the heat treatment.

Next, description will be made of a method of forming an oxide film on a metallic member and a method of cementing a metallic member according to a second embodiment of this invention.

In the oxide film forming method of the second embodiment, the oxide film is formed on the surface of a metallic member by treating the surface with a 34% hydrogen peroxide solution and thereafter silane (namely, γ-methacryloxy propyl triethoxy silane) is applied as a coupling agent on the oxidized surface of the metallic member, i.e., on the oxide film.

In the cementing method of the second embodiment, the oxide film is at first formed on the surface of the metallic member by treating the surface with 34% hydrogen peroxide solution and thereafter treating the oxidized surface with the silane coupling agent. Then, the metallic member is cemented through a resin-based adhesive to the fixing site of a patient as an affected part.

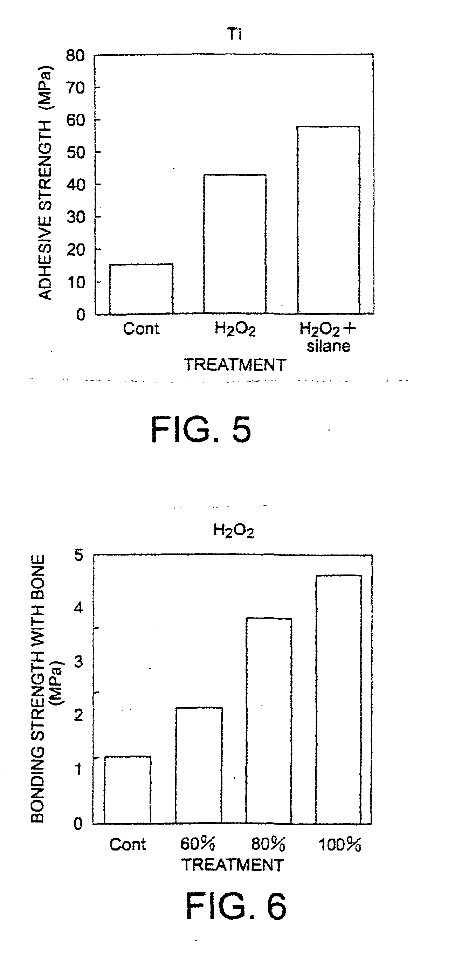

Referring to Fig. 5, the adhesive strength was measured in an experimental test for the second embodiment. Specifically, a titanium plate as the metallic member was treated with 34% hydrogen peroxide solution to form the oxide film, treated by a silane coupling agent, and cemented by a resin-based adhesive to another titanium plate similar treated. Then, the adhesive was measured in the manner similar to that mentioned in conjunction with Fig. 1. Preparation was also made of the control in which titanium plates without the treatment by the 34% hydrogen peroxide solution, i.e., without the oxide films were cemented to each other by the resin-based adhesive.

As illustrated in Fig. 5, the adhesive strength was 16 MPa in the control and was 42 MPa in the treatment by the 34% hydrogen peroxide alone. By a combination of the treatment by the hydrogen peroxide treatment and the treatment by the silane coupling agent, a most excellent adhesive strength of 58 MPa was achieved.

Referring to Fig. 6, the titanium implant was treated by the hydrogen peroxide solution in three different concentrations to form the oxide film on the surface of the implant in the manner similar to that in conjunction with the first embodiment. Specifically, the 34% hydrogen peroxide solution as a 100% starting solution was diluted to 60%, diluted to 80%, and not diluted. The titanium implant was dipped in the hydrogen peroxide solutions for several minutes. Thereafter, the implant was implanted to the tiba of the rabbit and, after lapse of eight weeks, the bonding strength with the bone was measured by the pull-out test.

The result is shown in Fig. 6. Preparation was also made of the control in which the implant without the hydrogen peroxide treatment, i.e., without the oxide film was implanted to the tiba of the rabbit.

From the result of measurement, the titanium implant treated by the hydrogen peroxide solution required a greater drawing force than that required in the titanium implant without such treatment, i.e., without the oxide film. Presumably, the bonding strength between the bone and the titanium implant was improved by the treatment with the hydrogen peroxide treatment. Thus, it has been revealed that the wettability of the bone tissue is improved by the oxide film formed by the treatment with the hydrogen peroxide solution.

As described above, according to the oxide film forming method of this invention, the oxide film similar to that obtained by the conventional heat treatment is formed on the surface of the metallic member within several minutes by the use of hydrogen peroxide alone or in combination with the silane coupling agent.

Thus, the metallic member need not be heated and oxidized in the electric furnace and oxidation can reliably be carried out in a short time.

According to the cementing method of this invention, the oxide film is formed on the surface of the metallic member by the use of hydrogen peroxide alone or in combination with the silane coupling agent and the metallic member cemented to the affected part through the cementing material.

As described above, the process form the oxidation to the cementing can be quickly and reliably carried out without requiring the heat treatment and the oxidation in the electric furnace,

According to the oxide film forming method and the cementing method of this invention, it is possible to avoid the deterioration in quality of the metallic member due to the high-temperature heat treatment and to save the time for polishing the surface of the metallic member.

## Claims

1. A method of forming an oxide film on a metallic member of a dental article, said method being for forming said oxide film on only a surface of a predetermined portion of said metallic member, said method comprising the step of
oxidizing the surface of said predetermined portion by the use of a hydrogen peroxide solution to form said oxide film.

2. A method as claimed in claim 1, further comprising the step of:
applying silane on said oxide film as a coupling agent.

3. A method as claimed in claim 1 or 2, wherein said metallic member is formed by a material selected from a group consisting of a cobalt-chromium alloy, a nickel-chromium alloy, stainless steel, pure titanium, a titanium alloy, a platinum gold alloy, a gold-silver-palladium alloy, a silver alloy, and a gold alloy.

4. A method as claimed in any of claim 1, 2 or 3, wherein said metallic member is an implant.

5. A method of cementing a metallic member, comprising the steps of:
oxidizing the surface of said predetermined portion by a hydrogen peroxide solution to form said oxide film;
applying a cementing material on the oxide film; and
cementing said predetermined portion to said affected part through the cementing material to fix the metallic member to the affected part.

6. A method as claimed in claim 5, further comprising the step of:
applying silane as a coupling agent to the oxide film before the cementing step.

7. A method as claimed in claim 5 or 6, wherein said metallic member is formed by a material selected from a group consisting of a cobalt-chromium alloy, a nickel-chromium alloy, stainless steel, pure titanium, a titanium alloy, a platinum gold alloy, a gold-silver-palladium alloy, a silver alloy, and a gold alloy, said cementing material being a dental cement.

8. A method as claimed in any of claims 5 to 8, wherein said metallic member is selected from a group including a cobalt-chromium alloy, a nickel-chromium alloy, stainless steel, pure titanium, a titanium alloy, a platinum gold alloy, a gold-silver-palladium alloy, a silver alloy, and a gold alloy, said cementing material being a resin-based adhesive.
